# EUROPEAN PATENT SPECIFICATION

(11) **EP 3 186 387 B1**
(45) Date of publication and mention of the grant of the patent: **17.10.2018**
(21) Application number: 15774495.4
(22) Date of filing: 25.08.2015
(51) Int. Cl.: C12Q 1/34

(54) **METHOD OF DETERMINING THE ACTIVITY OF ENZYMES CONVERTING CYTOSINE DERIVATIVES TO URACIL DERIVATIVES IN CELLS, TISSUES AND ORGANISMS**
VERFAHREN ZUR BESTIMMUNG DER AKTIVITÄT VON ENZYMEN ZUR UMWANDLUNG VON CYTOSINDERIVATEN IN URACILDERIVATE IN ZELLEN, GEWEBEN UND ORGANISMEN
PROCÉDÉ DE DÉTERMINATION DE L'ACTIVITÉ D'ENZYMES DE CONVERSION DE DÉRIVÉS DE CYTOSINE EN DÉRIVÉS D'URACILE DANS DES CELLULES, DES TISSUS ET DES ORGANISMES

(30) Priority: 26.08.2014 CZ 201400579 U
(43) Date of publication of application: 05.07.2017
(73) Proprietor: Univerzita Palackého v Olomouci, 771 47 Olomouc (CZ); Ustav organicke chemie a biochemie AV CR, v.v.i., 166 10 Praha 6 (CZ); Institute of Applied Biotechnologies, A.S., 101 00 Praha 10 (CZ)
(72) Inventor: KOBERNA, Karel, 779 00 Olomouc (CZ); LIGASOVA, Anna, 779 00 Olomouc (CZ); ROSENBERG, Ivan, 16500 Praha 6 (CZ); LIBOSKA, Radek, 252 63 Roztoky u Prahy (CZ); PAV, Ondrej, 197 00 Praha 9 (CZ); KVAPIL, Petr, 101 00 Praha 10 - Kolovraty (CZ); FISER, Josef, 312 00 Plzen (CZ)
(74) Representative: Hartvichova, Katerina
(86) International application number: PCT/CZ2015/000097
(87) International publication number: WO 2016/029889

(56) References cited:
- WO-A1-2009/021551
- CZ-B6- 303 252
- US-A1- 2004 059 104
- US-A1- 2011 020 799
- SANTOS O ET AL: "Radiation, pool size and incorporation studies in mice with 5-chloro-22-deoxycytidine", INTERNATIONAL JOURNAL OF RADIATION: ONCOLOGY BIOLOGY PHYSICS, PERGAMON PRESS, USA, vol. 19, no. 2, 1 August 1990 (1990-08-01) , pages 357-365, XP026847456, ISSN: 0360-3016 [retrieved on 1990-08-01]
- ALEX H. TUTTLE ET AL: "Immunofluorescent Detection of Two Thymidine Analogues (CldU and IdU) in Primary Tissue", JOURNAL OF VISUALIZED EXPERIMENTS, no. 46, 7 December 2010 (2010-12-07), XP055234559, DOI: 10.3791/2166
- DEZHONG QU ET AL: "5-Ethynyl-2-deoxycytidine as a new agent for DNA labeling: Detection of proliferating cells", ANALYTICAL BIOCHEMISTRY, ACADEMIC PRESS INC, NEW YORK, vol. 417, no. 1, 23 May 2011 (2011-05-23), pages 112-121, XP028245779, ISSN: 0003-2697, DOI: 10.1016/J.AB.2011.05.037 [retrieved on 2011-05-30]
- DAVID BRUST ET AL: "Radiosensitization of rat glioma with bromodeoxycytidine and adenovirus expressing herpes simplex virus-thymidine kinase delivered by slow, rate-controlled positive pressure infusion", CANCER GENE THERAPY, vol. 7, no. 5, 1 May 2000 (2000-05-01), pages 778-788, XP055234562, DOI: 10.1038/sj.cgt.7700168
- COOPER G M ET AL: "THE EFFECT OF INHIBITION OF CYTIDINE DEAMINASE BY TETRA HYDRO URIDINE ON THE UTILIZATION OF DEOXY CYTIDINE AND 5 BROMODEOXY CYTIDINE FOR DNA SYNTHESIS", MOLECULAR PHARMACOLOGY, vol. 9, no. 6, 1973, pages 698-703, XP9187586, ISSN: 0026-895X
- DEZHONG QU ET AL: "5-Ethynylcytidine as a new agent for detecting RNA synthesis in live cells by "click" chemistry", ANALYTICAL BIOCHEMISTRY., vol. 434, no. 1, 1 March 2013 (2013-03-01) , pages 128-135, XP055234676, NEW YORK. ISSN: 0003-2697, DOI: 10.1016/j.ab.2012.11.023
- KAISER I I ET AL: "Specific incorporation of 5-fluorocytidine into Escherichia coli RNA", BIOCHIMICA ET BIOPHYSICA ACTA . GENE STRUCTURE AND EXPRESSION, ELSEVIER, AMSTERDAM, NL, vol. 825, no. 1, 24 May 1985 (1985-05-24), pages 12-20, XP025221350, ISSN: 0167-4781, DOI: 10.1016/0167-4781(85)90074-0 [retrieved on 1985-05-24]
- David A Boothman ET AL: "Protective, Tumor-selective Dual Pathway Activation of 5-Fluoro-2'-deoxycytidine Provided by Tetrahydrouridine in Mice Bearing Mammary Adenocarcinoma-755", CANCER RESEARCH Epidemiology, and Public Health [T. V. BJCancer Res, vol. 47 1 May 1987 (1987-05-01), pages 2344-2353, XP055234687, Retrieved from the Internet: URL:http://cancerres.aacrjournals.org/cont ent/47/9/2344.full.pdf
- CASAFONT I ET AL: "Nuclear organization and dynamics of transcription sites in rat sensory ganglia neurons detected by incorporation of 5'-fluorouridine into nascent RNA", NEUROSCIENCE, NEW YORK, NY, US, vol. 140, no. 2, 1 January 2006 (2006-01-01), pages 453-462, XP027019515, ISSN: 0306-4522 [retrieved on 2006-05-17]

## Description

### Field of Art

The invention relates to the method of determining the activity of enzymes converting cytosine derivatives to uracil derivatives in cells, tissues and organisms.

### Background Art

Thanks to the activities of deaminases, transformations of a whole series of cytosine derivatives into uracil derivatives take place. Cytosine specific enzymes also participate in the deamination of some analogues of deoxycytidine and their monophosphates. An example of such analogues are some medicaments, e.g. ara-C (1-β-arabinofuranosylcytosine), dFdC (2',2'-difluoro-2'-deoxyuridine), PSI-6130 (β-D-2'-deoxy-2'-C-methylcytidine), L-dC (β-L-2'-deoxycytidine) or 5-aza-dC (5-aza-2'-deoxycytidine). It is expected that their deamination could influence the results of a treatment (e.g. Fundam Clin Pharm, 25, 172-185).

The most common approach for determining the activity of cytosine deaminases in cells is the analysis of the products of deamination after the disintegration of the cells, or tissues. However, when using current techniques, it is a relatively long process, often with the usage of radioactive markers, which allows the acquisition of data on the average activity in a relatively large population of cells, but not in small cell populations, or in individual cells (for a synopsis, see e.g. Fundam Clin Pharm, 25, 172-185; Cancer Res, 47, 3130-3135). In this respect, Santos et al. (International Journal of Radiation: Oncology, Biology, Physics, 19(2), 357-365) discloses a method of determining the activity of cytidine deaminase using 5-chloro-2'-deoxycytidine, which transforms it into 5-chloro-2'-deoxyuridine showing that the amount of 5-chloro-2'-deoxyuridine is proportional to the cytidine deaminase activity. The patent US 2011/020799 A1 discloses a kit comprising 5-ethynyl-2'-deoxycytidine or 5-ethynylcytidine, and a labelled azide, suitable for click reaction detection. In this respect, Dezhong Qu et al. (Analytical Biochemistry, 417, 112-121) shows ethynyl nucleotide derivatives and their detection in nucleic acids by click chemistry.Tuttle, A. H. et al. (Journal of Visualized Experiments, 46, e2166), describes detection of 5-chloro-2'-deoxyuridine in DNA using an antibody.

Cooper, G. M., et al. (Molecular Pharmacology, 9, 698-703) discloses a method of determining the activity of cytidine deaminase using 5-bromo-2'-deoxycytidine and detection of its deaminated product in DNA, testing the effect of the cytidine deaminase inhibitor tetrahydrouridine.

Brust D., et al. (Cancer Gene Therapy, 7, 778-788) teaches a method of measuring 5-bromo-2'-deoxyuridine incorporation in DNA, following exposure to 5-bromo-2'-deoxycytidine. The uridine derivative was detected using antibody.

Kaiser I.I. et al. (Biochimica et Biophysica Acta, 825, 12-20) discloses that 5-fluorocytidine is a substrate of cytidine deaminase in *Escherichia coli,* and that the resulting product - 5-fluorouridine - is incorporated in some species of RNA in *Escherichia coli.*

Currently, however, there is a lack of quick and reliable alternative procedures for determining the activity of cytosine deaminases, using different enzyme substrates, suitable for incorporation into DNA or RNA.

### Disclosure of the Invention

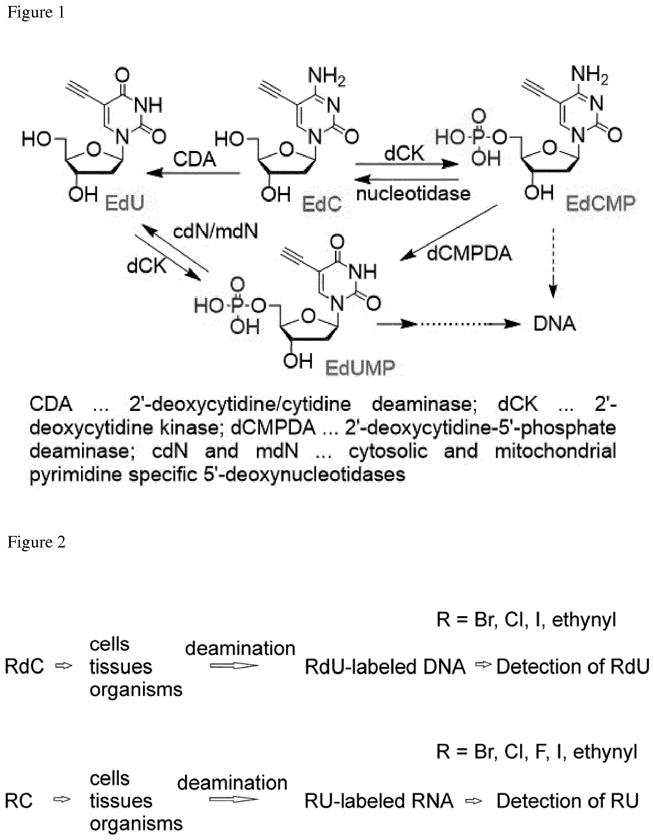

The present invention provides a method allowing a quick determination of the activity of enzymes transforming cytosine derivatives into uracil derivatives, preferably of the enzyme cytidine deaminase, in a sample of tissues, cells or in entire organisms, using 5-ethynyl-2'-deoxycytidine (EdC). This substance is transformed into its uracil derivative by deamination, and is subsequently detected in DNA. EdC is transformed into 5-ethynyl-2'-deoxyuridine (EdU, an example of a deaminases, which could participate in the deamination of EdU is in Fig. 1). The amount of the uracil nucleoside analogue is subsequently determined using a reaction with a specific antibody recognizing this nucleoside. In the case when EdC appear in the DNA only in an amount less than 10 % in comparison with the uracil derivative or are entirely lacking, the detection is preferably carried out using the so-called "click reaction" (Proc Natl Acad Sci U SA, 105, 2415-2420), which is, in comparison with the antibody detection, very quick and highly effective.

When the invention is used in systems, which contain cytidine deaminase (CDA) and deoxycytidine 5' -monophosphate deaminase (dCMP deaminase) as the dominant deamination enzymes, both deaminases participate in the transformation of EdC. Although the signal is produced by both enzymes, the measured signal is mainly produced by the enzyme of cytidine deaminase in the absolute majority of the common natural systems, according to our findings. In most cases, it is therefore possible to use both groups of nucleosides for a precise determination of the CDA activity in these systems.
For the analysis of the signal produced by the antibodies or a click reaction, it is possible to use, i.e. classical microscopic techniques or techniques based on the use of devices of the type of flow cytometers or plate readers.
Preferably, for increasing the incorporation of uracil derivatives, mainly of EdU into DNA, 5-fluoro-2'-deoxyuridine (FdU) is used. In order to determine the deaminase activity when using EdC, the sample is incubated at the same time with an addition of 5-fluoro-2'-deoxyuridine. This step significantly increases the signal without a substantial influence on the deamination activity of the cellular enzymes, and thus allows a determination of the deamination activity also in samples, which have an extremely low ability to incorporate uracil derivatives. In order to increase the signal, it is usually sufficient to use a concentration in the order of units to tens of nmol.l⁻¹.

Preferably, the samples are fixed before the reaction with an antibody, e.g. using formaldehyde, methanol itself or ethanol itself or methanol and subsequently acetone.
The scheme of the determination of the nucleosides is depicted in Fig. 2. For determining of the deamination activity, preferably the curve of dependence of the size of the signal on the concentration of the uracil and cytosine analogues is used. For a comparison of the deamination activity, the value of the signal of the cytosine analogue, which is under the saturation values, is preferably used. The saturation values are understood to be the values, where there is no longer an increase of the signal upon further increase of the concentration of the cytosine analogue. From the calculated or measured signal for this value, the value of the concentration of the uracil analogue, which corresponds to this signal, is then determined. This is how to determine the production of the uracil analogue, which is produced by the cytosine analogue of a given concentration. This value is then a reflection of the deamination activity (see Example 1).

The sample can be, for instance, a sample of a tissue or cells or an entire organism (e.g. a laboratory animal).
The present invention further provides the use of the method of determining the activity of the enzymes transforming cytosine derivatives into uracil derivatives, preferably of the enzyme of cytidine deaminase, for determining the deaminase activity in the cells treated with substances, which influence the deaminase activity of the enzymes transforming cytosine derivatives into uracil derivatives. Such substances could be small molecules, e.g. diazepinone ribonucleoside, 3,4,5,6-tetrahydrouridine or zebularine, substances that are potentially suitable as new inhibitors of deaminases, or oligonucleotide sequences of DNA or RNA (e.g. siRNA) targeted to the inhibition of the expression of the above mentioned deaminases.

The application of the invention in laboratory animals *in vivo* can be used for example in testing of substances potentially suitable as new deaminase inhibitors, or testing of oligonucleotide sequences of DNA or RNA (e.g. siRNA) targeted to the inhibition of the expression of the above mentioned deaminases. Moreover, it can be utilized in situations when deamination of a medicament can influence the results of the treatment.

### Brief Description of the Drawings

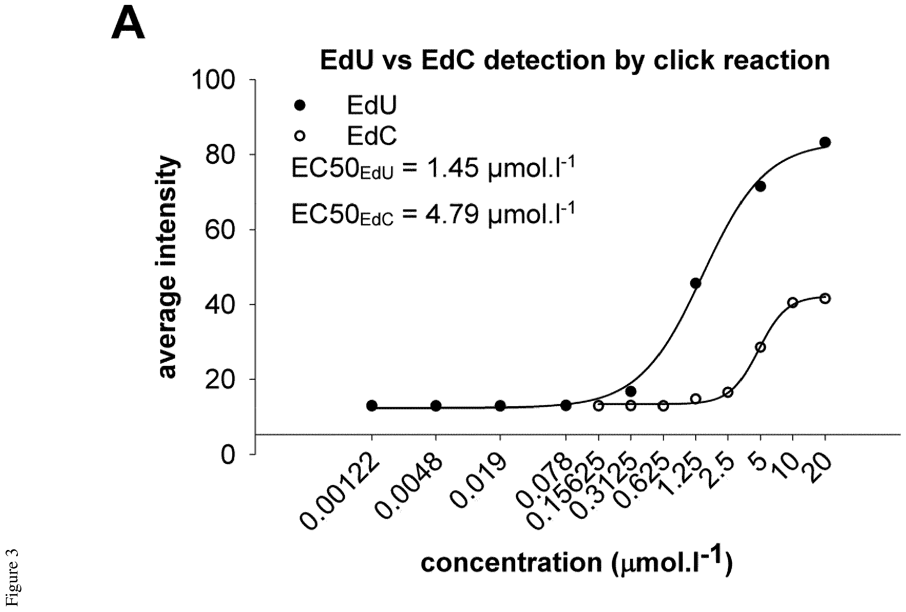

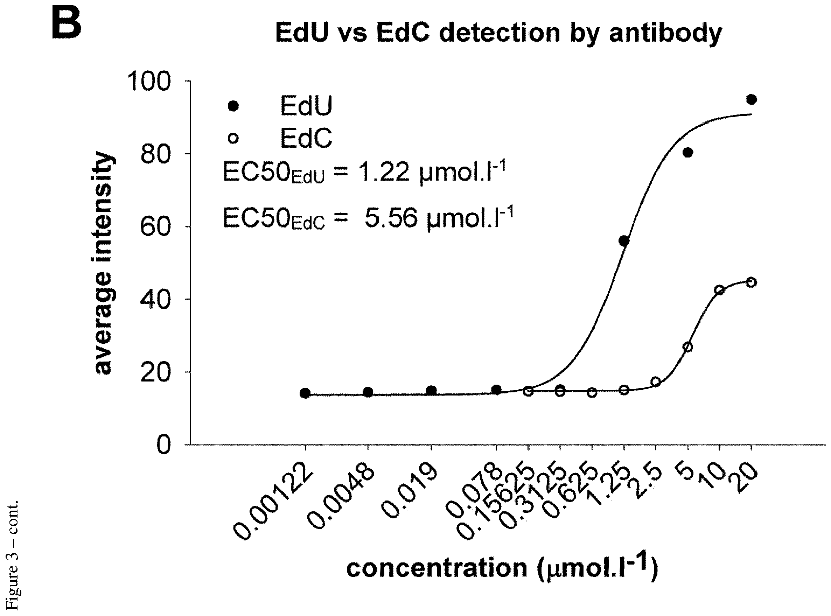

**Figure 1** The metabolism of EdC and EdCMP from the perspective of the deamination activity of some known deaminases in human cells.
   CDA - 2'-deoxycytidine/cytidine deaminase; dCK - 2'-deoxycytidine kinase; dCMPDA - 2'-deoxycytidine-5'-phosphate deaminase; cdN and mdN - cytosolic and mitochondrial pyrimidine specific 5'-deoxynucleotidase
**Figure 2****:** Schematic depiction of determination of the activity of the enzymes transforming cytosine derivatives into uracil derivatives.
**Figure 3****:** Determination of the deamination activity in HeLa cells using specific antibodies or using a click reaction (Example 1).
   The figure depicts the dependence of the average intensity of the signals on the used concentrations of EdU or EdC after two hours of incubation with various concentrations of EdU or EdC without the addition of FdU. Two methods were used for the detection of both modified nucleosides: A) detection with a click reaction with an azido dye (Alexa Fluor 488- azide); B) detection using primary anti-bromodeoxyuridine antibody and secondary antibody conjugated with Alexa Fluor 488. The measured intensities of the signals were processed in the Sigma Plot software, in which the curve was constructed and the half of maximal effective concentration (EC50) was determined. It is clear from the course of the graphs that EdC is not likely to be incorporated in the DNA. This was confirmed also by the mutual ratios between the measured values of the intensities obtained by the antibodies and by the click reaction. These ratios were 1.099 for EdU and 1.060 for EdC. The value of ratios of EC50 in EdU and EdC (K_{EdU½}/K_{EdC½}) was 0.23 for the detection performed by the antibodies and 0.32 for the detection performed by the click reaction. According to our findings from the analysis of the isolated DNA, the content of EdC in DNA was less than 10%, which would correspond to similar results obtained by two independent methods. It is clear from the course of the dependence of the signal on the used concentrations of EdC that it is also possible to use, preferably, the value of the concentration of EdC, e.g. around 5 µmol.l⁻¹, for the comparison of the deamination activity. This value provides approximately such a signal as 0.55 µmol.l⁻¹ EdU and is not in the saturation area. For the determination, it is also possible to use other measured concentrations, because there was no saturation of the signal even at higher concentrations.

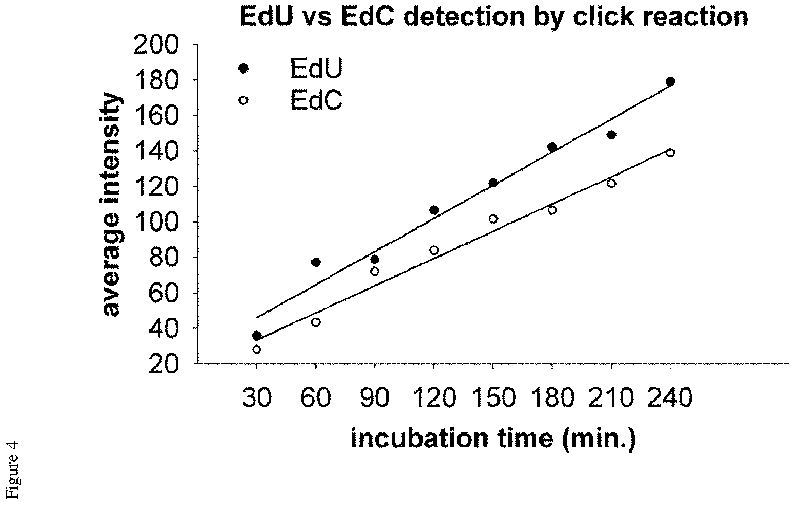

**Figure 4** Time curve (Example 1).
   The time course of the incorporation of EdU and EdC into DNA of HeLa cells. In this case, the cells were incubated with 10 µmol.l⁻¹ EdU or EdC for a period of 30, 60, 90, 120, 150, 180, 210 and 240 minutes. The incorporated nucleosides were detected using a click reaction with azidofluorochrome.

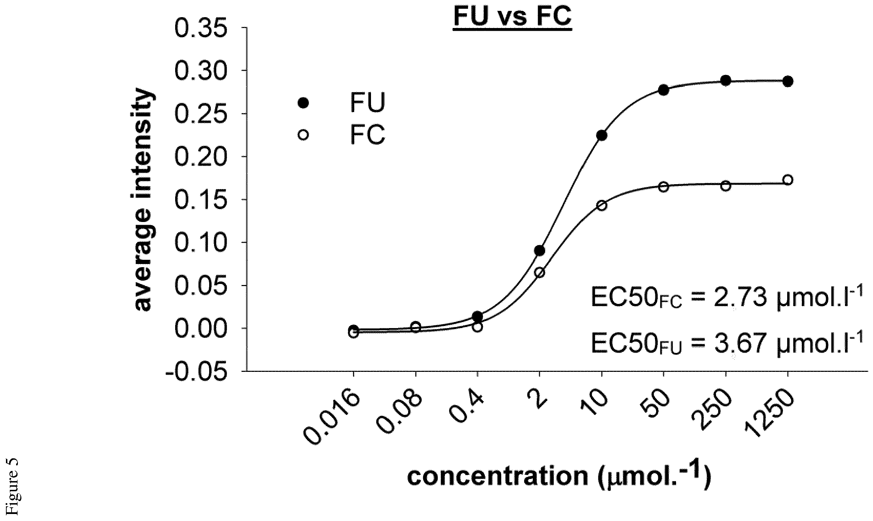

**Figure 5****:** The comparison of the incorporation of FU and FC into HeLa cells when using various concentrations (Example 1).
   The cells were incubated for a period of 2 hours in a medium with various concentrations of FU or FC. The same method was used as in Fig. 3.

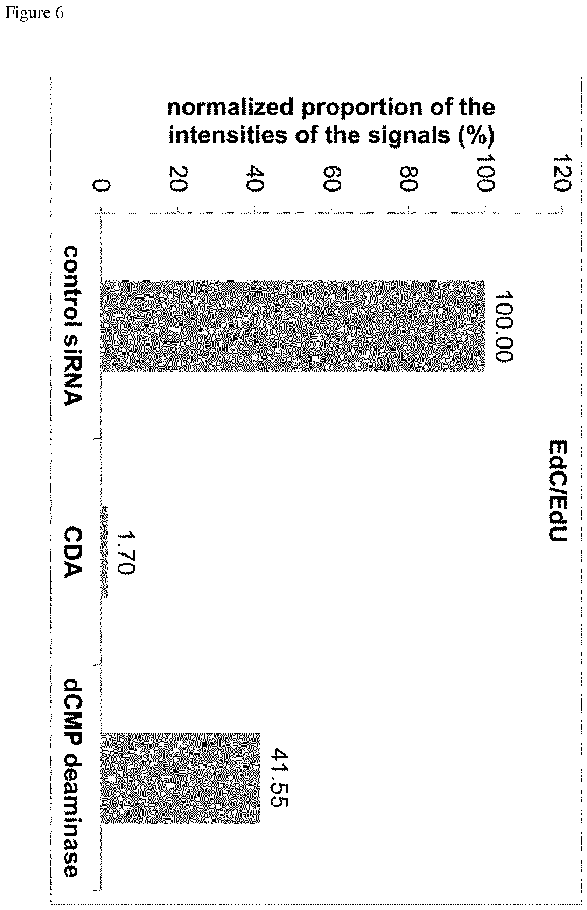

**Figure** 6 The effect of siRNA, targeted against CDA and dCMP deaminase, on the intensity of the signals of EdU and EdC in HeLa cells (Example 7).
   The cells influenced by siRNA, targeted against CDA or dCMP deaminase, were incubated for 2 hours with 10 µmol.l⁻¹ of EdU or EdC. EdU was detected with antibodies. The normalized ratio of the average intensities of the signals of EdC/EdU after the treatment of cells with siRNA, targeted against CDA, dCMP deaminase, and control siRNA is depicted in the resulting graph. Although the signal decreased also in the cells influenced by siRNA against dCMP deaminase, our measurement demonstrated that in this case there was a rapid decrease also of CDA. This decrease relatively precisely corresponded to the decrease of the signal of EdC/EdU. The data shown are in percentages. 100% corresponds to the ratio for the control cells influenced by the control, inactive siRNA.

### Examples

### List of abbreviations used

HeLa cell line of human epithelial cells
DMEM Dulbecco's modified Eagle's medium
PBS Phosphate-buffered saline
S-MEM Spinner modification-Eagle's minimum essential medium for suspension cells
All of the examples are based on the transformation of individual cytosine derivatives into uracil derivatives and the subsequent determination of the content of these derivatives in DNA or RNA using an antibody or a click reaction with azido molecules (Fig. 2).

### Example 1

Determining the activity of the enzymes transforming cytosine derivatives into uracil derivatives in adherent cells on slides.

The following approach describes the method of determining the activity of the enzymes transforming cytosine derivatives into uracil derivatives in adherent HeLa cells on slides. Unless mentioned otherwise, all of the steps were performed at room temperature, whereas the steps followed one another without any further time delays.
A) Human HeLa cells (hereinafter referred to only as samples) were cultivated in Petri dishes on circular coverslips with a diameter of 12 mm in a growth medium - DMEM with L-glutamine (Gibco), 10% (vol./vol.) Fetal Bovine Serum (PAA Laboratories), 50 µg/ml gentamicine and 3.7 g/l NaHCO₃ in an atmosphere with 5% (vol./vol.) CO₂ at 37 °C.
B) On the second day after the passage, the following nucleosides were added to the growth medium alternatively 0.00064; 0.0032; 0.016; 0.08; 0.4; 2; 10; 20; 50; 250; 1 250 or 6 250 µmol.l⁻¹ EdC or EdU for a period of 30, 60, 90, 120, 150, 180, or 210 minutes or 4 or 6 or 24 hours. In some cases, FdU was simultaneously added in the concentration of 1, 10 or 1000 nmol.l⁻¹ with the addition of EdC or EdU.
C) The growth medium was subsequently removed and replaced by a buffer (1x PBS, for the composition, see the section Solutions and chemicals used, approximately 1 to 2 ml of the buffer was added on a 4 cm² surface).
D) The buffer was removed and the samples were fixed for 10 minutes in a 2% (weight/vol.) solution of formaldehyde in 1x PBS (pH 7.4; 1 to 2 ml of the fixation solution on a 4 cm² surface).
   Alternatively, the samples were fixed with methanol and subsequently acetone. In this case, the coverslips with the samples were first incubated for 10 minutes in methanol at a temperature of -20 °C (approximately 1 to 2 ml of methanol on a 4 cm² surface) and subsequently were submerged for 30 seconds in a beaker with 50 ml of acetone at room temperature, the coverslips with the samples were then dried and after drying placed in a Petri dish with 1x PBS, where the samples were turned upward to the solution.

In some cases, methanol or ethanol alone was used for the fixation. The coverslips with the samples were incubated in 50-100% ethanol of methanol (approximately 1 to 2 ml on a 4 cm² surface) for a period of 10 minutes at room temperature and the coverslips with the samples were subsequently dried and after drying placed in a Petri dish with 1x PBS, where the samples were turned upward to the solution.
In the case of the use of methanol, ethanol or methanol and subsequently acetone, step G) followed.
E) The solution of formaldehyde was removed and the samples were covered with 1x PBS (approximately 1 to 2 ml 1x PBS on a 4 cm² surface). After 5 minutes, the buffer was removed and replaced with a new one of the same composition. This step was repeated one more time.
F) The buffer was subsequently removed. The samples were permeabilized with a 0.2% (vol./vol.) solution of Triton X-100 in 1x PBS (approximately 1 to 2 ml of the permeabilization solution on a 4 cm² surface) for 10 minutes. After removing the permeabilization solution, the samples were covered with 1x PBS (approximately 1 to 2 ml 1x PBS on a 4 cm² surface).
G) After 5 minutes, the solution of 1x PBS was removed and replaced with the same amount of the same solution. This step was repeated one more time.
H) In the case that antibodies were used for the detection of EdU, 1x PBS was removed and replaced with an aqueous solution of hydrochloric acid (4 mol.l⁻¹, approximately 1 to 2 ml of the acid solution on a 4 cm² surface). This step serves to reveal EdU in the structure of DNA. Alternatively, any other method revealing EdU in the structure of DNA can be used. It concerns practically all of the described methods used for revealing BrdU (see, e.g., PloS One, 7, e52584 or Genes Dev, 14, 2855-2868 or J Cell Sci, 115, 4037-4051). After 20 minutes of incubation at room temperature, the hydrochloric acid solution was removed and replaced by 1x PBS.
   In the other cases, step H was left out and work continued with step J.
I) After 5 minutes, the solution of 1x PBS was removed and replaced with the same amount of the same solution. This step was repeated one more time.
J) The coverslips with the samples were removed from the Petri dish using tweezers and the rest of the buffer was drawn off using filtration paper so that the coverslips with the samples were placed on the side without the samples on the filtration paper. The coverslips with the samples were placed on drops of 1x PBS on a parafilm (150 µl/drop, this size was always used unless it is mentioned otherwise). The coverslips were orientated with the layer with the samples down onto the drops. If EdU or EdC was detected using azidofluorochromes, the experiment moved to step N. In the other cases, the experiment continued with step K.
K) The samples were incubated for 30 minutes on 20 µl drops with an antibody recognizing EdU but not EdC, diluted in 1x PBS (the concentration of the antibody was 5 µg/ml, hereinafter referred to only as primary antibody).
L) The coverslips with the samples were transferred to a drop of 1x PBS. After 5 minutes, the coverslips with the samples were transferred to a new drop of 1x PBS of the same composition. This step was repeated three times.
M) The coverslips with the samples were incubated for 30 minutes with an antibody conjugated with fluorochrome Cy3 (secondary antibody) and reacting with the primary antibody on drops of a size of 20 µl (the secondary antibody was diluted in a proportion of 1:100 in 1x PBS, the final concentration of the antibody was 5 µg/ml). The experiment subsequently continued according to step O.
N) Subsequently, the rest of the 1x PBS was drawn off the samples using filtration paper, so that the filtration paper was gently placed on the edge of the coverslip and the coverslips with the samples were moved onto a 50 µl drop of the solution for the conjugation of the azidofluorochrome with EdU/EdC in the presence of copper ions (a click reaction). The buffer contained 100 mmol.l⁻¹ of Tris-Cl, pH 7.4, 10 mmol.l⁻¹ sodium ascorbate, 4 mmol.l⁻¹ copper(II) sulphate and 20 µmol.l⁻¹ tetramethylrhodamine-azide (TAMRA-azide). The samples were incubated on this mixture for 20 minutes.
O) The coverslips with the samples were then transferred to a drop of 1x PBS. After 5 minutes, the coverslips with the samples were moved to a new drop of 1x PBS of the same composition. This step was repeated three more times. The coverslips were then transferred to a drop of 100 mmol.l⁻¹ of Tris-Cl and 100 mmol.l⁻¹ of NaCl, pH 7.5. This step was repeated two more times, the coverslips were subsequently transferred to a drop of 10 µmol.l⁻¹ of DAPI in 100 mmol.l⁻¹ of Tris-Cl and 100 mmol.l⁻¹ of NaCl, pH 7.5 and incubated on the drop for a period of 20 minutes. Subsequently, the coverslips with the samples were moved to a drop of distilled water. After 5 minutes, the coverslips with the samples were moved to a new drop of distilled water.
P) In the end, the water was drawn off the coverslips using filtration paper, the coverslips were placed on a new filtration paper with the samples up and dried.
Q) After drying, the coverslips were placed on drops (3 µl) of the prepared mounting medium on a slide (solution: 90% glycerol, 50 mM Tris, pH 8, 2.5% 1,4-diazabicyclo[2.2.2]octane). The samples were placed into a drop of the solution. With gentle pressure using the tweezers on the upper side of the coverslips with the samples, the solution of the medium spread over the entire area of the coverslips. Subsequently, the edges of the coverslips were lacquered using a quick-drying lacquer. The signal was detected using a fluorescence microscope. The example of determining the deaminase activity in HeLa cells using EdC and EdU is depicted in Figs. 3 and 4, and using FC and FU in Fig. 5.

### Example 2

Determining the activity of the enzymes transforming cytosine derivatives into uracil derivatives in tissues.

The following approach describes the method of determining the activity of the enzymes transforming cytosine derivatives into uracil derivatives in liver tissues of three-day-old rats. Unless mentioned otherwise, all of the steps were performed at room temperature, whereas the steps followed one another without any further time delays.
A) Three-day-old male rats were killed by decapitation and their livers were subsequently removed. The livers were cut into small pieces (3 mm - the longest dimension) and placed in Petri dishes with HeLa cells incubated for one day in the growth medium DMEM with L-glutamine (Dulbecco's modified Eagle's medium, Gibco), 10% (vol./vol.) of Fetal Bovine Serum (PAA Laboratories), 50 µg/ml of gentamicine and 3.7 g/l of NaHCO₃. Into the medium with the pieces of liver, the following nucleosides were added 0.00064; 0.0032; 0.016; 0.08; 0.4; 2; 10; 20; 50; 250; 1 250 or 6 250 µmol.l⁻¹ of EdC or EdU for a period of 30, 60, 90, 120, 150, 180, or 210 minutes or 4 or 6 or 24 hours.
B) The growth medium was subsequently removed and the pieces of the liver were covered by a buffer (1x PBS, for the composition, see the section Solutions and chemicals used, approximately 1 to 2 ml of the buffer was added on a 4 cm² surface). After 5 minutes, the buffer was removed and replaced with the same amount of 1x PBS of the same composition. This step was repeated one more time.
C) The pieces of liver were places between two circular coverslips with a diameter of 12 mm coated with 1% (weight/vol.) of gelatine and the tissues were spread over the surface of the coverslips using a gentle pressure of the tweezers on the coverslip. The coverslips were separated and both were placed in Petri dishes with 1x PBS, so that the coverslips orientated the tissue samples up towards the solution.
D) The following steps of the experiment were performed according to the steps described in Example 1 (D-Q).

### Example 3

Determining the activity of the enzymes transforming cytosine derivatives into uracil derivatives in a cellular suspension.

The following approach describes the method of determining the activity of the enzymes transforming cytosine derivatives into uracil derivatives in suspensions of HeLa S3 cells. Unless mentioned otherwise, all of the steps were conducted at room temperature, whereas the steps followed one another without any further time delays.
A) Human HeLa S3 cells (hereinafter referred to only as samples) were grown in cultivation flasks (the volume of the flask was 50 ml), which were shaken, in the growth medium S-MEM (Spinner modification-Eagle's minimum essential medium for the suspension of cells, Sigma) with 3% (weight/vol.) of L-glutamine, 5% (vol./vol.) of Fetal Bovine Serum (PAA Laboratories) and 50 µg/ml of gentamicine in an atmosphere with 5% (vol./vol.) of CO₂ at 37 °C. The cellular suspension contained approximately 5-8 x 10⁵ cells per 1 millilitre.
B) The second day after the passage, 0.00064; 0.0032; 0.016; 0.08; 0.4; 2; 10; 20; 50; 250; 1 250 or 6 250 µmol.l⁻¹ of EdC or EdU were added to the growth medium for a period of 30, 60, 90, 120, 150, 180, or 210 minutes or 4 or 6 or 24 hours.
C) Subsequently, the suspension of the cells was transferred to a 15-millilitre centrifuge test tube and the samples were centrifuged for 10 minutes at 300*g*. The supernatant was removed (200 µl of the medium was left in the test tube) and a new medium (10 ml) was added to the samples. The samples were re-suspended and subsequently centrifuged again for 10 minutes at 300*g*. The supernatant was removed (200 µl of the medium was left in the test tube) and 1 ml of new media was added to the pellet. The pellet of the samples was re-suspended.
D) A 50-µl drop of the cell suspension was transferred to coverslips with a diameter of 12 mm coated by a layer of poly-L-lysine (the method of the preparation of these coverslips is described in the section Solutions and chemicals). After 2 minutes, the excess of the medium was draw off using filtration paper, so that the filtration paper was gently placed on the edge of the coverslip. The coverslips were placed in 2% (weight/vol.) of a formaldehyde solution in a Petri dish and the samples were fixed for 10 minutes. The coverslips were placed in the Petri dish so that the samples were orientated upward in the solution.
E) In the following steps, the experiment was performed according to steps E-Q of Example 1.

The proposed method of the detection of deaminase activity can be used also when working with cell suspensions for the entire period of their processing, for instance for FACS (Fluorescence Activated Cell Sorting). In this case, the experiment was performed according to steps A-C of Example 3, and subsequently according to steps D-O of Example 1 whereas during removals of all of the solutions, additions of new solutions and rinsing, the cells were centrifuges for 10 minutes at 300*g*, the supernatant was subsequently removed (200 µl of the solution was left in the test tube), the new solution was added to the cells according to the Example 1 (2 ml), the cells were re-suspended in it and incubated for the period listed in the individual steps of Example 1. In step O, the cells were not rinsed with water, they were coated with 2 ml of 1x PBS instead. For measurements of the fluorescence intensity, a flow cytometer was used.

### Example 4

Determining the activity of the enzymes transforming cytosine derivatives into uracil derivatives in fixed cells when using cultivation plates.

The method is the same as in the case of the adherent cells (Example 1, steps A-O) with the following differences:
1) All of the incubations were conducted in the wells of 96-well plates.
2) In incubations with antibodies or with azidofluorochromes, 50 µl of the solution was used.
3) In the case of rinsing, 200 µl of the solution was used.
4) At the end of the protocol (step O of Example 1), water is removed from the individual samples and is replaced with 100 µL of a mixture of 90% of glycerol, 50 mM of Tris, pH 8, 2.5% 1,4-diazabicyclo[2.2.2]octane.
5) For fluorescence measurements, besides the microscopic measurement, measurements using microplate readers were used as well.
In the case of the use of microplate readers, increasing the signal using a reaction with alkaline phosphatase or peroxidase was the most preferable embodiment. In these cases, azido-biotin was used instead of azidofluorochromes and antibodies conjugated with alkaline phosphatase or peroxidase instead of antibodies conjugated with fluorochrome. In the case of the use of azido-biotin, streptavidin conjugated with alkaline phosphatase or peroxidase after a triple rinsing with 200 µl of 1x PBS was used for its detection. The incubation with streptavidin was performed in the same way as the incubation with the secondary antibody according to step M of Example 1. The concentration of streptavidin was 1 µg/ml. After the incubation of the samples with streptavidin or the antibody with a peroxidase or alkaline phosphatase, the samples were rinsed three times with 200 µl of 1x PBS and 100 µl of the substrate was added to the samples. In the case of the use of alkaline phosphatase, 1-Step PNPP (Thermo Scientific) was used as a substrate, in the case of the peroxidase, 1-Step™ Turbo TMB-ELISA (Thermo Scientific) was used as a substrate and everything was performed according to the instructions of the producer.

### Example 5

Determining the activity of the enzymes transforming cytosine derivatives into uracil derivatives in cells grown in suspension using cultivation plates.

The method was the same as in the case of the cell suspensions processed for FACS (Example 3) with the following differences:
1) Human HeLa S3 cells (hereinafter referred to only as samples) were grown in wells of plastic cultivation microplates (200 µl of cellular suspension in each well) in the growth medium S-MEM (Spinner modification-Eagle's minimum essential medium for suspension cells) with 3% (weight/vol.) of L-glutamine, 5% (vol./vol.) of Fetal Bovine Serum (PAA Laboratories) and 50 µg/ml of gentamicine in an atmosphere with 5% (vol./vol.) of CO₂ at 37 °C. The cellular suspension contained approximately 1 x 10⁵ cells per 1 millilitre.
2) The samples were centrifuged for 5 minutes at 300*g*.
3) All of the incubation were conducted in wells.
4) For rinsing, 200 µl of the solution was used.
5) In incubations with antibodies or with azido molecules, 50 µl of the solution was used.
6) The measurements were conducted in a solution of 90% of glycerol, 50 mM of Tris, pH 8, 2.5% of 1,4-diazabicyclo[2.2.2]octane.
7) For the measurement of fluorescence, measurement using a microscope or microplate reader was used.

### Example 6

Determining the activity of the enzymes transforming cytosine derivatives into uracil derivatives in cells influenced by the inhibitors of cytidine deaminase (CDA, EC 3.5.4.5) diazepinone ribonucleoside or 3,4,5,6-tetrahydrouridine or zebularine in cells and tissues.

The method was the same as in Examples 1-5 with the following changes:
The cells were incubated at the same time with the nucleosides and 10 µmol.l⁻¹ of diazepinone ribonucleoside or 3,4,5,6 tetrahydrouridine or zebularine (Cancer Chemoth and Pharm, 30, 7-11).

### Example 7

Determining the activity of the enzymes transforming cytosine derivatives into uracil derivatives in cells and tissues treated with siRNA targeted against CDA or dCMP deaminase.

The method was the same as in Examples 1, 3, 4 and 5 with the following changes:
1) The cells were grown in the case of the fixed cells either on 12-mm circular coverslips in Petri dishes or in 96-well microplates, in the case of suspension cells in cultivation test tubes with a volume of 15 ml or in 96-well microplates. In all the cases, they were grown in a complete cultivation medium without antibiotics.
2) The cells were placed so that the cells on the second day reached 60-80% density. In fixed cells, 100% density means that the area was entirely filled with cells.
3) The second day after the passage, the cultivation medium was removed. In the case of cell suspensions grown in cultivation test tubes, the samples were first centrifuged for 10 minutes at 300*g* and the supernatant was subsequently removed. The suspension cultures were similarly first centrifuged in the further steps before the solution was exchanged.
4) The samples were subsequently rinsed with transfection medium (purchased along with solutions of siRNA).
   In the case of cultivation on coverslips in the Petri dishes, 1-2 ml of the transfection medium was used on a 4 cm² surface. In the case of cell suspensions grown in cultivation test tubes, after the removal of the supernatant, 2 ml transfection medium were added to the samples and they were re-suspended. In the case of cultivation in 96- well cultivation microplates, 100 µl of transfection medium was used for each well.
5) The transfection medium was removed.
   The siRNA solution (for the composition, see the section Solutions and chemicals used) was subsequently added to the samples. In the case of cultivation on coverslips in Petri dishes, 1.5 ml of siRNA solution for 8 cm² of area was added. In the case of cell suspensions grown in cultivation test tubes, after the removal of the supernatant, 2.5 ml of siRNA solution was added to the samples and the samples were re-suspended. In the case of cultivation in 96-well cultivation microplates, 100 µl of siRNA solution was used for 1 well. The cells were incubated for 7 hours in a CO₂ incubator at 37 °C and 5% of CO₂.
6) A cultivation medium, which contained a double amount of the fetal bovine serum and antibiotic than is used in the medium during common cultivations of cells, was subsequently added to the samples. In the case of cultivation on coverslips in Petri dishes, 1.5 ml of medium was added for 8 cm² of area. In the case of cell suspensions grown in cultivation test tubes, 2.5 ml of the medium was added. In the case of cultivation in 96-well cultivation microplates, 100 µl of the medium was added for 1 well. The siRNA solution was not removed. The cells were incubated for 24 hours in a CO₂ incubator at 37 °C and 5% of CO₂.
7) The medium was subsequently removed.
8) The common cultivation medium was added to the samples. In the case of cultivation on coverslips in Petri dishes, 2 ml of the medium was added for 8 cm² of area. In the case of cell suspensions grown in cultivation test tubes, 3 ml of the medium was added to the samples after the removal of the supernatant and the samples were re-suspended. In the case of cultivation in 96-well cultivation microplates, 100 µl of the medium was used for 1 well. The cells were incubated for 24 hours in a CO₂ incubator at 37 °C and 5% CO₂.
9) Subsequently, the following nucleosides were added to the growth medium alternatively: 0.00064; 0.0032; 0.016; 0.08; 0.4; 2; 10; 50; 250; 1 250 or 6 250 µmol.l⁻¹ of EdC or EdU for a period of 20 minutes or 2 hours or 6 hours or 24 hours.
Subsequently, the experiment was conducted according to Examples 1, 3, 4 or 5. The example determining the deaminase activity after the influence of the siRNA targeted against CDA or dCMP deaminase is in Fig. 6.

### Example 8

Determining the activity of the enzymes transforming cytosine derivatives into uracil derivatives in the cells and tissues of mice.

The method was the same as in Examples 1 and 2 with the following changes:
1. Live mice were injected with 0.1 or 1 or 10 or 100 mg of EdC or EdU in PBS and after 8 hours the mouse was killed by decapitation, its tissues cut into small pieced and placed in PBS.

### Solutions and chemicals used:

### 1. Phosphate-buffered saline (PBS)

10x PBS (a ten times concentrated solution of PBS):
1.4 mol.l⁻¹ of NaCl
26 mmol.l⁻¹ of KCl
90 mmol.l⁻¹ of Na₂HPO₄ 14 mmol.l⁻¹ of KH₂PO₄

The listed components were dissolved in distilled water and the pH was adjusted to the range of 7.3-7.4
1x PBS (phosphate-buffered saline in the usual concentration)

### 2. Preparation of poly-lysine coverslips for anchoring the suspension cells

Circular coverslips with a diameter of 12 mm were placed in a 50 ml beaker with 20 ml of 96% (vol./vol.) ethanol. After 10 minutes, they were transferred using a tweezers into a Petri dish lined with filtration paper and dried. The coverslips were subsequently submerged for a period of 1 minute into a 0.01% (weight/vol.) solution of poly-L-lysine in DMEM, subsequently submerged for 30 seconds into 1x PBS and then once again for 30 seconds into a new 1x PBS. This step was repeated 3 more times. In the end, the coverslips were placed on filtration paper and dried.

### 3. siRNA solution - composition

**Solution A:** 0.5 mM siRNA targeted against CDA or dCMP deaminase and transfection medium (Santa Cruz Biotechnologies).

**Solution B:** the transfection solution was diluted with the transfection medium according to the instructions of the provider (Santa Cruz Biotechnologies).

The two prepared solutions were mixed so that Solution A was added to Solution B, stirred with a thin pipette and left to incubate at room temperature for 15-45 minutes. Subsequently, 4 parts of the transfection media was added to the solution, the whole solution was stirred and added to the samples.

### Industrial Applicability

The method of determining the activity of the enzymes transforming cytosine derivatives to uracil derivatives in cells and tissues can be used, for instance, (i) in seeking of new inhibitors of deaminases or (ii) testing various cell lines from the perspective of deaminase activities or (iii) in the diagnostics related with the application of medicaments based on cytosine analogues of nucleosides or (iv) in testing the effect of DNA or RNA oligonucleotides (antisense oligonucleotides and siRNA) targeted at the inhibition of the expression of the above mentioned deaminases.

## Claims

1. A method of determining deaminase activity of enzymes transforming cytosine derivatives into uracil derivatives in a tissue or cell *in vitro* or in a non-diagnostic method in an entire organism of a laboratory animal, **characterized in that** the tissue or cell is incubated or the laboratory animal is injected with 5-ethynyl-2'-deoxycytidine, wherein 5-ethynyl-2'-deoxycytidine is transformed in the presence of the enzyme into deaminated product: 5-ethynyl-2'-deoxyuridine, and 5-ethynyl-2'-deoxyuridine is subsequently detected in DNA, and the amount of the incorporated 5-ethynyl-2'-deoxyuridine in the DNA is determined.

2. The method according to claim 1, wherein the amount of incorporated 5-ethynyl-2'-deoxyuridine in the DNA is determined using a reaction with an antibody.

3. The method according to claim 2, **characterized in that** the deaminated product is subsequently detected in DNA of fixed samples, respectively.

4. The method according to claim 1, where the amount of incorporated 5-ethynyl-2'-deoxyuridine in the DNA is determined using a click reaction.

5. The method according to any one of the claims 1, 3 and 4 for determination of deaminase activity using 5-ethynyl-2'-deoxycytidine, wherein the sample is simultaneously incubated with 5-fluoro-2'-deoxyuridine.

6. Use of the method according to any one of the claims 1 to 5 for determination of deaminase activity in cells treated with substances which influence the deaminase activity of the enzymes transforming cytosine derivatives into uracil derivatives.

## Patentansprüche

1. Verfahren zur Bestimmung der Deaminaseaktivität von Enzymen, die Cytosinderivate in Uracilderivate in einem Gewebe oder einer Zelle *in vitro* oder in einem nichtdiagnostischen Verfahren in einem gesamten Organismus eines Labortieres transformieren, **dadurch gekennzeichnet, dass** das Gewebe oder die Zelle inkubiert wird, oder das Labortier wird injiziert, mit 5-Ethinyl-2'-Desoxycytidin, wobei 5-Ethinyl-2'-Desoxycytidin in Gegenwart des Enzyms in desaminiertes Produkt 5-Ethinyl-2'-Desoxyuridin umgewandelt wird, und das 5-Ethinyl-2'-Desoxyuridin anschließend in DNA nachgewiesen wird und die Menge an eingebautes 5-Ethinyl-2'-desoxyuridin in die DNA wird bestimmt.

2. Verfahren nach Anspruch 1, wobei die Menge an eingebautem 5-Ethinyl-2'-desoxyuridin in der DNA unter Verwendung einer Reaktion mit einem Antikörper bestimmt wird.

3. Verfahren nach Anspruch 2, **dadurch gekennzeichnet, dass** das desaminierte Produkt anschließend in DNA von fixierten Proben detektiert wird.

4. Verfahren nach Anspruch 1, wobei die Menge an eingebautem 5-Ethinyl-2'-desoxyuridin in der DNA unter Verwendung einer Klick-Reaktion bestimmt wird.

5. Verfahren nach einem der Ansprüche 1, 3 und 4 zur Bestimmung der Deaminaseaktivität unter Verwendung von 5-Ethinyl-2'-Desoxycytidin, wobei die Probe gleichzeitig mit 5-Fluor-2'-desoxyuridin inkubiert wird.

6. Verwendung des Verfahrens nach einem der Ansprüche 1 bis 5 zur Bestimmung der Desaminaseaktivität in Zellen, die mit Substanzen behandelt sind, die die Deaminaseaktivität der Enzyme beeinflussen, die Cytosinderivate in Uracilderivate umwandeln.

## Revendications

1. Un procédé de détermination de l'activité désaminase d'enzymes transformant des dérivés de cytosine en dérivés d'uracile dans un tissu ou dans une cellule *in vitro* ou dans une méthode non diagnostique dans un organisme entier d'un animal de laboratoire, **caractérisée en ce que** le tissu ou la cellule est incubé, ou l'animal de laboratoire est injecté, avec 5-éthynyl-2'-désoxycytidine, dans lequel 5-éthynyl-2'-désoxycytidine est transformée en présence de l'enzyme en un produit désaminé: 5-éthynyl-2'-désoxyuridine, et 5-éthynyl-2'-désoxyuridine est ensuite détecté dans l'ADN et la quantité de 5-éthynyl-2'-désoxyuridine incorporée dans l'ADN est déterminée.

2. Le procédé selon la revendication 1, dans lequel la quantité de 5-éthynyl-2'-désoxyuridine incorporée dans l'ADN est déterminée en utilisant une réaction avec un anticorps.

3. Le procédé selon la revendication 2, **caractérisé en ce que** le produit désaminé est ensuite détecté dans l'ADN d'échantillons fixes, respectivement.

4. Le procédé selon la revendication 1, dans lequel la quantité de 5-éthynyl-2'-désoxyuridine incorporée dans l'ADN est déterminée en utilisant une réaction clic.

5. Le procédé selon l'une quelconque des revendications 1, 3 et 4 pour détermination de l'activité désaminase en utilisant la 5-éthynyl-2'-désoxycytidine, dans lequel l'échantillon est simultanément incubé avec 5-fluoro-2'-désoxyuridine.

6. Utilisation du procédé selon l'une quelconque des revendications 1 à 5 pour détermination de l'activité désaminase dans des cellules traitées avec des substances qui influencent l'activité désaminase des enzymes transformant les dérivés de cytosine en dérivés d'uracile.
